# EUROPEAN PATENT APPLICATION

(11) **EP 2 213 243 A1**
(43) Date of publication of application: **04.08.2010**
(21) Application number: 08845371.7
(22) Date of filing: 29.10.2008
(51) Int. Cl.: A61B 10/00, A61Q 17/04, G01N 21/65

(54) **EFFECTIVENESS EVALUATION METHOD, EFFECTIVENESS EVALUATION DEVICE, AND EFFECTIVENESS EVALUATION PROGRAM ALL FOR EXTERNAL PREPARATION FOR SKIN**

(30) Priority: 02.11.2007 JP 2007286607
(71) Applicant: Shiseido Company, Ltd., Chuo-ku Tokyo 104-8010 (JP)
(72) Inventor: IWAKI, Haruhi, Yokohama-shi Kanagawa 224-8558 (JP); EGAWA, Mariko, Yokohama-shi Kanagawa 224-8558 (JP)
(74) Representative: Kügele, Bernhard
(86) International application number: PCT/JP2008/069684
(87) International publication number: WO 2009/057657

(57) **Abstract**

An effectiveness evaluating method evaluates effectiveness of an external preparation for skin for protection against ultraviolet irradiation after the external preparation is applied to the skin. The method includes a measurement step of measuring an amount of urocanic acid in a horny cell layer by Raman spectroscopy both before and after the application of the external preparation and both before and after exposure to ultraviolet irradiation, an index derivation step of computing at least one index for evaluating the effectiveness of the external preparation based on results of the measurement obtained in the measurement step, and an evaluation step of evaluating the effectiveness of the external preparation by using the index obtained in the index derivation step.

## Description

### TECHNICAL FIELD

This invention relates to an effectiveness evaluating method, an effectiveness evaluating device, and an effectiveness evaluating program for an external preparation for skin. In particular, this invention relates to an effectiveness evaluating method, an effectiveness evaluating device, and an effectiveness evaluating program which are adapted for simply and speedily evaluating the effectiveness of an external preparation for skin for protection against ultraviolet irradiation, based on an index derived from a depth distribution of urocanic acid in the skin measured by Raman spectroscopy.

### BACKGROUND ART

With the recent progress of photobiology and optical medicine, it has become evident that adverse effects or chronic changes in skin on a human body induced by repeated ultraviolet irradiation of the skin over an extended period of time may be causes of diseases, such as a cell damage, photodermatosis, skin cancer or a cataract, and may result in the photo-ageing of skin, such as a spot, a crease, etc.

It is known that acute changes in the skin induced by ultraviolet irradiation may include sunburn, suntan, and local or systemic hypofunction of the immune system. About the carcinogenesis induced by ultraviolet irradiation, a possibility of promoting the carcinogenesis is pointed out because of the carcinogenicity of ultraviolet irradiation itself and the falling of skin immunoreaction induced by ultraviolet irradiation.

In recent years, it has been known widely that the adverse effects of ultraviolet irradiation on the skin, include the falling of skin immunoreaction, the photo-ageing of skin, such as a spot, a crease, a benign or malignant tumor of the skin, etc., and it has been recognized that protection against ultraviolet irradiation by using an external preparation for skin having an ultraviolet irradiation protective effect is very important for protection against photo-ageing.

Even in weather in which an external preparation having an ultraviolet irradiation protective effect is not used daily, continued exposure of skin to ultraviolet irradiation every day may lead to the photo-ageing of the skin, and there is concern about various adverse effects of ultraviolet irradiation on a living body.

Conventionally, known indexes of an ultraviolet irradiation protective effect for a sunscreen or protector which is an external preparation for skin with an ultraviolet irradiation protective effect (or a sunscreen effect) are the Sun Protection Factor SPF which employs the erythema as the index, or the PA (Protection grade of UVA) indication which is ranked by using the PFA (Protection Factor of OVA) value. In such situations, if the osmotic effect of ultraviolet irradiation into skin can be represented by using a certain index, it will make it possible to take effective measures for protection against ultraviolet irradiation.

The reaction of skin induced by ultraviolet irradiation may be represented by a photochemical reaction, and this photochemical reaction is initiated by the chromophore on the side of a living body that absorbs light energy. On the other hand, light energy is absorbed by intracellular nucleic acid, protein, or other molecules. The molecules are excited by absorption of light energy, resulting in various changes in the cell functions. Examples of the major chromophore in skin may include DNA (deoxyribonucleic acid) in an epidermic cell and urocanic acid in a horny cell layer.

DNA has an absorption maximum wavelength near 260 nm and absorbs ultraviolet light with wavelengths in a UVB range (the wavelength: 290 nm - 320 nm) directly, which may cause the DNA injuries, such as thymine dimer, (6-4)photoproduct, etc.

Urocanic acid (4-imidazoleacrylic acid) is a water-soluble ultraviolet absorption substance made by deamination of histidine within keratinocyte (cornification cell) and exists mostly in a horny cell layer. Isomerization of trans-urocanic acid to cis-urocanic acid takes place when exposed to ultraviolet irradiation (mainly, UVB). The comparison of wavelengths of UV acting on the immunosuppression (mice) and the wavelengths of the photoreceptor in skin, and the result of tape stripping showing UCA (urocanic acid) as the causative substance were reported in 1983 by De Fabo et al. (for example, see Non-Patent Document 1 below). Thereafter, the involvement of UCA (urocanic acid) in the immunosuppression by ultraviolet irradiation has been studied by many labs.

It has become clear from these results that cis-urocanic acid is one of the factors of the immunosuppression by ultraviolet irradiation, but the detailed mechanism is not known yet. It is considered that trans-urocanic acid works as the chromophore within a horny cell layer and defends for the inside of the skin so that the adverse effects of ultraviolet irradiation may not affect the inside of the skin.

Conventionally, Raman spectroscopy has been used as a technique for analyzing the components in the tissue of a cell of a living body (for example, see Patent Document 1 below). The use of Raman spectroscopy enables the measurement of a distribution or profile of moisture or an amino acid in skin or a medicine applied to skin in the depth direction (for example, see Non-Patent Document 2 below).

Non-Patent Document 1: E. C. De Fabo and F. P. Noonan "Mechanism of Immune Suppression by Ultraviolet Irradiation in Vivo, I. Evidence for the Existence of a Unique Photoreceptor in Skin and its Role in Photoimmunology", J. Exp. Med. vol. 157, 1983, 84-98

Patent Document 1: Japanese Laid-Open Patent Publication No. 2006-508358

Non-Patent Document 2: Peter J. Caspers, Gerald W. Lucassen, Elizabeth A. Carter, Hajo A. Bruining, and Gerwin J. Puppels "In Vivo Confocal Raman Microspectroscopy of the Skin: Noninvasive Determination of Molecular Concentration Profiles", J. Invest. Dermatol. vol. 116, 2001, 434-442

### DISCLOSURE OF THE INVENTION

### PROBLEM TO BE SOLVED BY THE INVENTION

However, the relationship between the effectiveness of an external preparation for skin for ultraviolet irradiation protection and the depth distribution of the isomerized urocanic acid in the horny cell layer by ultraviolet irradiation is not studied adequately. There is no method to evaluate the effectiveness of the external preparation for skin for protection against ultraviolet irradiation, based on the depth distribution of the isomerized urocanic acid in the horny cell layer by ultraviolet irradiation which can be measured by Raman spectroscopy.

In an aspect of the invention, the present disclosure provides an effectiveness evaluating method, an effectiveness evaluating device, and an effectiveness evaluating program which are able to simply and speedily evaluate the effectiveness of an external preparation for skin for protection against ultraviolet irradiation, based on an index derived from a depth distribution of urocanic acid in the skin measured by Raman spectroscopy.

### MEANS FOR SOLVING THE PROBLEM

In an embodiment of the invention which solves or reduces one or more of the above-mentioned problems, the present disclosure provides an effectiveness evaluating method which evaluates effectiveness of an external preparation for skin for protection against ultraviolet irradiation after the external preparation is applied to the skin, the method including: a measurement step of measuring an amount of urocanic acid in a horny cell layer by Raman spectroscopy both before and after the application of the external preparation and both before and after exposure to ultraviolet irradiation; an index derivation step of computing at least one index for evaluating the effectiveness of the external preparation, based on results of the measurement obtained in the measurement step; and an evaluation step of evaluating the effectiveness of the external preparation by using the at least one index obtained in the index derivation step. It is possible for the present disclosure to simply and speedily evaluate the effectiveness of a UV protective external preparation for skin based on the index derived from the depth distribution of the amount of urocanic acid in the skin measured by Raman spectroscopy.

The effectiveness evaluating method may be arranged so that the measurement step measures a depth distribution of the amount of urocanic acid in the horny cell layer both before and after the application of the external preparation. The conventional methods have to extract urocanic acid from the skin and measure the amount of urocanic acid outside the skin. However, the present disclosure can measure the amount of urocanic acid within the skin and can detect the isomerization of urocanic acid at a certain depth in the skin in the same position before and after exposure to UV irradiation. The present disclosure does not require the time for processing of samples and thus makes it possible to evaluate the effectiveness of the external preparation for protection against UV irradiation simply and speedily.

The effectiveness evaluating method may be arranged so that the index derivation step computes at least one of a total amount of urocanic acid in the horny cell layer, an average amount of urocanic acid in the horny cell layer, a depth distribution of the amount of urocanic acid in the horny cell layer from a skin surface, a residual ratio of urocanic acid in the horny cell layer, and a total amount of urocanic acid at three surface points in the depth distribution, as the at least one index based on the results of the measurement. One of the plural indexes computed by using the results of the measurement of the amount of urocanic acid in the horny cell layer can be selected for the evaluation and therefore the effectiveness of the UV protective external preparation can be simply and speedily evaluated.

In an embodiment of the invention which solves or reduces one or more of the above-mentioned problems, the present disclosure provides an effectiveness evaluating device which evaluates effectiveness of an external preparation for skin for protection against ultraviolet irradiation after the external preparation is applied to the skin, the effectiveness evaluating device including: a measurement part to measure an amount of urocanic acid in a horny cell layer by Raman spectroscopy both before and after the application of the external preparation and both before and after exposure to ultraviolet irradiation; an index derivation part to compute at least one index for evaluating the effectiveness of the external preparation, based on results of the measurement obtained by the measurement part; and an evaluation part to evaluate the effectiveness of the external preparation by using the at least one index obtained by the index derivation part. It is possible for the present disclosure to simply and speedily evaluate the effectiveness of a UV protective external preparation for skin based on the index derived from the depth distribution of the amount of urocanic acid in the skin measured by Raman spectroscopy.

The effectiveness evaluating device may be arranged so that the measurement part measures a depth distribution of the amount of urocanic acid in the horny cell layer both before and after the application of the external preparation. The conventional methods have to extract urocanic acid from the skin and measure the amount of urocanic acid outside the skin. However, the present disclosure can measure the amount of urocanic acid within the skin and can detect the isomerization of urocanic acid at a certain depth in the skin in the same position before and after exposure to UV irradiation. The present disclosure does not require the time for processing of samples and thus makes it possible to evaluate the effectiveness of the external preparation for protection against UV irradiation simply and speedily.

The effectiveness evaluating device may be arranged so that the index derivation part computes at least one of a total amount of urocanic acid in the horny cell layer, an average amount of urocanic acid in the horny cell layer, a depth distribution of the amount of urocanic acid in the horny cell layer from a skin surface, a residual ratio of urocanic acid in the horny cell layer, and a total amount of urocanic acid at three surface points in the depth distribution, as the at least one index based on the results of the measurement. One of the plural indexes computed by using the results of the measurement of the amount of urocanic acid in the horny cell layer can be selected for the evaluation and therefore the effectiveness of the UV protective external preparation can be simply and speedily evaluated.

In an embodiment of the invention which solves or reduces one or more of the above-mentioned problems, the present disclosure provides an effectiveness evaluating program which evaluates effectiveness of an external preparation for skin for protection against ultraviolet irradiation after the external preparation is applied to the skin, and, when executed by a computer, causes the computer to perform: a measurement step of measuring an amount of urocanic acid in a horny cell layer both before and after the application of the external preparation by Raman spectroscopy, which amount has changed by exposure to ultraviolet irradiation; an index derivation step of computing at least one index for evaluating the effectiveness of the external preparation, based on results of the measurement obtained in the measurement step; and an evaluation step of evaluating the effectiveness of the external preparation by using the at least one index obtained in the index derivation step. It is possible to simply and speedily evaluate the effectiveness of a UV protective external preparation for skin based on the index derived from the depth distribution of the amount of urocanic acid in the skin measured by Raman spectroscopy. Installation of the program enables a general-purpose personal computer to perform the effectiveness evaluating method of the present disclosure.

### EFFECTS OF THE INVENTION

According to the present disclosure, the effectiveness of an external preparation for skin having an ultraviolet irradiation protective effect can be simply and speedily evaluated based on an index derived from a depth distribution of an amount of urocanic acid in the skin measured by Raman spectroscopy.

### BRIEF DESCRIPTION OF THE DRAWING

FIG. 1 is a block diagram illustrating the composition of an effectiveness evaluating device of an embodiment of the invention.
FIG. 2 is a diagram illustrating the hardware composition of an effectiveness evaluating device of an embodiment of the invention for performing an effectiveness evaluating method which evaluates the effectiveness of an external preparation for skin for protection against ultraviolet irradiation.
FIG. 3 is a flowchart for explaining the effectiveness evaluating method of the present embodiment which evaluates the effectiveness of an external preparation for skin for protection against ultraviolet irradiation.
FIG. 4 is a diagram for explaining Example 1 of the index derivation and evaluation in the present embodiment.
FIG. 5 is a diagram for explaining Example 2 of the index derivation and evaluation in the present embodiment.
FIG. 6 is a diagram for explaining Example 3 of the index derivation and evaluation in the present embodiment.
FIG. 7 is a diagram illustrating an example of the evaluation result by Example 1 of the effectiveness evaluating method.
FIG. 8 is a diagram illustrating an example of the evaluation result by Example 2 of the effectiveness evaluating method.
FIG. 9 is a diagram illustrating an example of the evaluation result by Example 3 of the effectiveness evaluating method.

### DESCRIPTION OF REFERENCE NUMERAL

- 10: effectiveness evaluating device
- 11: input part
- 12: output part
- 13: storing part
- 14: measurement part
- 15: index derivation part
- 16: evaluation part
- 17: control part
- 21: input device
- 22: output device
- 23: drive device
- 24: auxiliary memory device
- 25: memory device
- 26: CPU
- 27: network connection device
- 28: recording medium

### BEST MODE FOR CARRYING OUT THE INVENTION

### <Outline of the Invention>

The present disclosure provides an index derived from an amount of urocanic acid existing in a skin horny cell layer and being isomerized by ultraviolet irradiation, in order to provide the mechanism for simply and speedily measuring and quantifying the adverse effects of ultraviolet irradiation on skin, and thereby simply and speedily evaluating the ultraviolet irradiation protective effect of an external preparation for skin.

In the following, a description will be given of an effectiveness evaluating method, an effectiveness evaluating device, and an effectiveness evaluating program for an external preparation for skin of embodiments of the invention with reference to the accompanying drawings.

### <Effectiveness Evaluating Device: Composition and Functions>

The composition of an effectiveness evaluating device of an embodiment of the invention will be described with reference to the accompanying drawings.

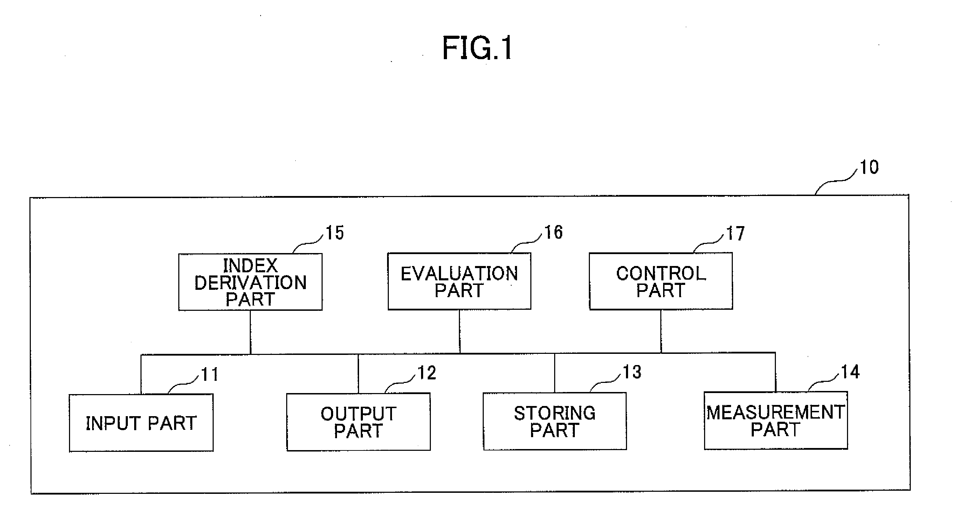

FIG. 1 is a block diagram illustrating the composition of an effectiveness evaluating device of an embodiment of the invention. As illustrated in FIG. 1, the effectiveness evaluating device 10 includes an input part 11, an output part 12, a storing part 13, a measurement part 14, an index derivation part 15, an evaluation part 16, and a control part 17.

The input part 11 receives various instructions, input by a user, such as measurement instructions for measuring an amount of urocanic acid in the skin using Raman spectroscopy, derivation instructions for deriving an index based on the measured amount of urocanic acid, and evaluation instructions for evaluating the effectiveness of the external preparation based on the derived index. For example, the input part 11 includes a keyboard and a pointing device, such as a mouse. The input part 11 is capable of inputting various kinds of data, including depth distributions of the amount of urocanic acid in a portion of the evaluation object before and after application of the external preparation to the skin, and depth distributions of the amount of urocanic acid in the portion of the evaluation object before and after exposure to ultraviolet irradiation, which data have been measured by an external device or the like.

The output part 12 performs display indication and outputting of the data input from the input part 11, and performs display indication and outputting of data as a result of computation based on the input data. The output part 12 includes a display unit, a speaker, etc. The output part 12 may have a function of a printer. In such a case, a result of the evaluation of the effectiveness can be printed on a printing medium, such as paper, so that the printing medium can be supplied to a user or the like.

The storing part 13 stores various kinds of data, such as a measurement result obtained by the measurement part 14, a derivation result obtained by the index derivation part 15, and an evaluation result based on the index and obtained by the evaluation part 16. The storing part 13 can read out the data stored therein, if needed.

The measurement part 14 measures an amount of urocanic acid existing in a skin horny cell layer in a portion of an evaluation object and being isomerized by ultraviolet irradiation, by using Raman spectroscopy. Specifically, the measurement part 14 measures a depth distribution of the amount of urocanic acid in the horny cell layer by using Raman spectroscopy. The amount of urocanic acid measured denotes an amount of cis-urocanic acid, or a ratio of an amount of trans-urocanic acid to an amount of cis-urocanic acid.

For example, in order to measure a depth distribution of the amount of urocanic acid in a horny cell layer, the measurement part 14 of this embodiment repeatedly performs the measurement of the amount of urocanic acid in the portion of the evaluation object at each of intervals of a predetermined depth from the skin surface (for example, for every two micrometers, for every five micrometers, or for every 10 micrometers) both before and after application of a UV protective external preparation (which is used in the effectiveness evaluating method) to the skin. Moreover, by using Raman spectroscopy, the measurement part 14 of this embodiment repeatedly performs the measurement of the depth distribution of each amount of urocanic acid in the portion of the evaluation object before and after application of the external preparation to the skin both before and after exposure to ultraviolet irradiation.

For example, the measurement using Raman spectroscopy may be performed by the composition of the instrument as disclosed in Patent Document 1 listed above. Alternatively, the measurement may be performed by using a different composition than disclosed in Patent Document 1 listed above. A description thereof will be given later.

The index derivation part 15 computes at least one of a total amount of urocanic acid in the horny cell layer, an average amount of urocanic acid in the horny cell layer, a depth distribution of the amount of urocanic acid in the horny cell layer from a skin surface, a residual ratio of urocanic acid in the horny cell layer, and a total amount of urocanic acid at three surface points in the depth distribution as the index based on the measured amounts of urocanic acid in the horny cell layer obtained from the measurement part 14. Namely, at least one of the computations selectively obtained by the index derivation part 15 is used as the index for evaluation of the effectiveness of the external preparation for skin for protection against ultraviolet irradiation.

In this manner, the amount of urocanic acid in the horny cell layer and the depth distribution thereof measured by Raman spectroscopy are used, and the adverse effect of ultraviolet irradiation on the skin before and after application of the external preparation to the skin can be evaluated simply and speedily.

The evaluation part 16 evaluates the effectiveness of the external preparation for protection against ultraviolet irradiation. Specifically, using the depth distributions of the amount of urocanic acid both before and after application of the external preparation to the skin and the depth distributions of the amount of urocanic acid both before and after exposure to ultraviolet irradiation, both obtained from the measurement part 14, and using at least one index obtained from the results of the measurement by the index derivation part 15, the evaluation part 16 performs the comparison of the depth distributions of the amount of urocanic acid in the horny cell layer, and evaluates the effectiveness of the external preparation for protection against ultraviolet irradiation based on the comparison result.

The control part 17 controls each of the component parts of the effectiveness evaluating device 10. Specifically, the control part 17 performs control for each of the step of measurement of the amount of urocanic acid, the index derivation step, the evaluation step, etc., in accordance with the instructions input from the input part 11 by the user.

### <Composition of Measurement Part 14>

Next, the composition of the measurement part 14 mentioned above will be described. The measurement part 14 of this embodiment measures a depth distribution of the amount of urocanic acid in the horny cell layer by Raman spectroscopy.

For example, the measurement of an amount of urocanic acid in a horny cell layer by Raman spectroscopy may be performed by using an instrument and a measuring method as disclosed in the Patent Document 1 mentioned above. In this case, the instrument including a laser, a signal detecting unit for measuring a Raman signal of an measurement object, an optical fiber probe having optical fibers, etc. may be used.

For example, in the measuring method using the above-mentioned instrument, a Raman signal of a measurement object is generated. Laser light is directed through an optical fiber to the measurement object, light that is scattered by the measurement object is collected through an optical fiber, the collected light is guided away from the measurement object to the signal detecting unit, and the Raman signal is detected by the signal detecting unit. Using an optical fiber having substantially no Raman signal in one or more parts of the 400-3700 cm⁻¹ spectral region, laser light is directed through the optical fiber to the measurement object, light that is scattered by the measurement object is collected through the optical fiber, the collected light is guided away from the measurement object to the signal detecting unit, and the Raman signal is detected by the signal detecting unit.

Namely, a laser beam is directed through the optical fiber to a portion of the evaluation object, and a Raman signal scattered by the portion is collected through the optical fiber and guided to the signal detecting unit, so that the Raman signal is detected by the signal detecting unit.

In the present embodiment, in order to measure a depth distribution of an amount of urocanic acid in a horny cell layer, the measurement of the amount of urocanic acid is performed at each of intervals of a predetermined depth (for example, 2 micrometers) from the skin surface in a predetermined range of a portion of the evaluation object both before and after application of the external preparation used in the effectiveness evaluating method.

### <Hardware Composition of Effectiveness Evaluating Device 10>

The above-described effectiveness evaluating device 10 may be provided by preparing a computer-executable program (effectiveness evaluating program) which, when executed by a computer, causes the computer to perform each of respective steps of the effectiveness evaluating method, and installing the program in a general-purpose personal computer, a server, etc. The effectiveness evaluating method for evaluating the effectiveness of an external preparation for skin for protection against ultraviolet irradiation can be carried out by the computer in which the effectiveness evaluating program is installed.

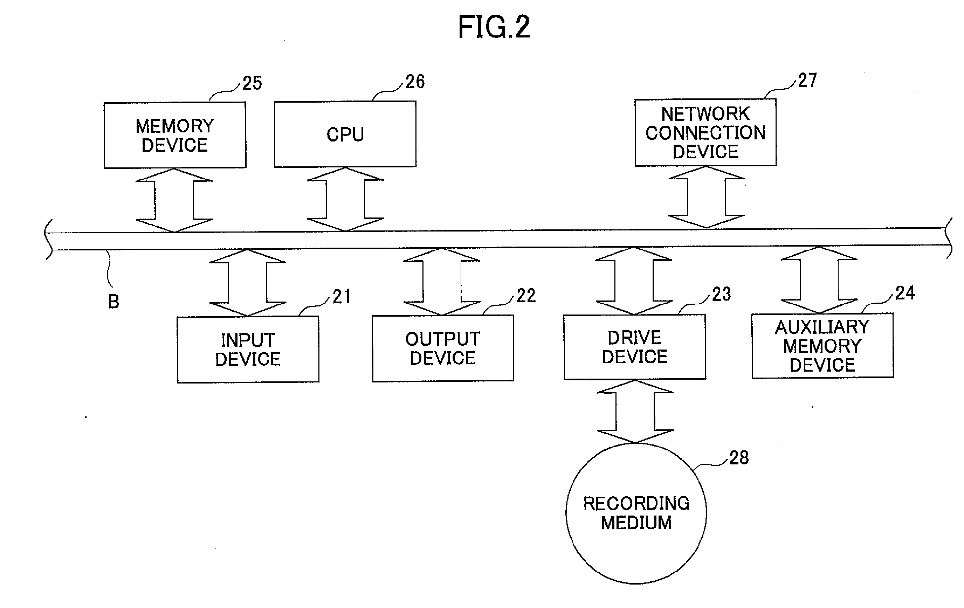

Next, the hardware composition of a computer of this embodiment which is adapted for performing the effectiveness evaluating method which evaluates the effectiveness of an external preparation for skin for protection against ultraviolet irradiation will be described. FIG. 2 is a diagram illustrating the hardware composition of an effectiveness evaluating device of this embodiment for performing the effectiveness evaluating method which evaluates the effectiveness of an external preparation for skin for protection against ultraviolet irradiation.

As illustrated in FIG. 2, the computer of this embodiment includes an input device 21, an output device 22, a drive device 23, an auxiliary memory device 24, a memory device 25, a CPU (Central Processing Unit) 26 that performs various kinds of control, and a network connection device 27. These elements of the computer are interconnected by a system bus B.

The input device 21 includes a keyboard and a pointing device, such as a mouse, which are operated by a user or the like. The input device 21 inputs various operation signals, such as an execution signal for execution of the program, input by the user or the like. The input device 21 is capable of inputting various kinds of data, including depth distributions of the amount of urocanic acid in the already measured portion of the evaluation object both before and after application of the external preparation to the skin and depth distributions of the amount of urocanic acid in the already measured portion of the evaluation object both before and after exposure to ultraviolet irradiation, which data are acquired from an external device connected to the network connection device 27 via the communication network.

The output device 22 includes a display unit which displays various windows and various kinds of data needed to operate the computer of this embodiment for performing the effectiveness evaluating method, and the display unit is capable of displaying the progress or the result of execution of the effectiveness evaluating program in accordance with the control program executed by the CPU 26. The output device 22 is capable of printing a result of the evaluation of the effectiveness on a printing medium, such as paper, so that the printing medium can be supplied to a user or the like.

For example, the computer executable program which is installed in the computer may be supplied by a removable recording medium 28, such as a USB (universal serial bus) memory, a CD-ROM, a DVD, etc. The recording medium 28 in which the computer executable program is stored can be set in the drive device 23, and the program stored in the recording medium 28 is installed from the recording medium 28 to the auxiliary memory device 24 through the drive device 23.

The auxiliary memory device 24 is a storage part, such as a hard disk. The auxiliary memory device 24 is capable of accumulating, inputting and outputting the computer executable program of the invention and other control programs provided in the computer, if needed.

The memory device 25 stores the computer executable program read from the auxiliary memory device 24 by the CPU 26. The memory device 25 may include a ROM (read only memory), a RAM (random access memory), etc.

In accordance with the control programs, such as OS (operating system), and the computer executable program stored in the memory device 25, the CPU 26 controls various tasks of processing of the entire computer, such as various computations and the inputting/outputting of data to and from the respective hardware component parts, and carries out each processing task. A variety of information needed during execution of the program may be acquired from the auxiliary memory device 24, and the auxiliary memory device 24 may also store a result of the execution of the program.

The network connection device 27 which is connected to the communication network is capable of receiving the computer executable program from an external device connected to the communication network and is capable of supplying to the external device the computer executable program of the invention and a result of execution of the program.

The network connection device 27 is capable of receiving, from the external device connected to the communication network, various kinds of data, including depth distributions of the amount of urocanic acid in the portion of the evaluation object both before and after application of the external preparation to the skin and both before and after exposure to ultraviolet irradiation, which data have been already measured by the external device.

By using the above-described hardware composition of the computer, the effectiveness evaluating method which evaluates the effectiveness of an external preparation for skin for protection against ultraviolet irradiation can be performed. The effectiveness evaluating method which evaluates the effectiveness of an external preparation for skin for protection against ultraviolet irradiation can be carried out by a general-purpose personal computer in which the program is installed.

### <Effectiveness Evaluating Method for External Preparation for Skin for Protection against Ultraviolet Irradiation>

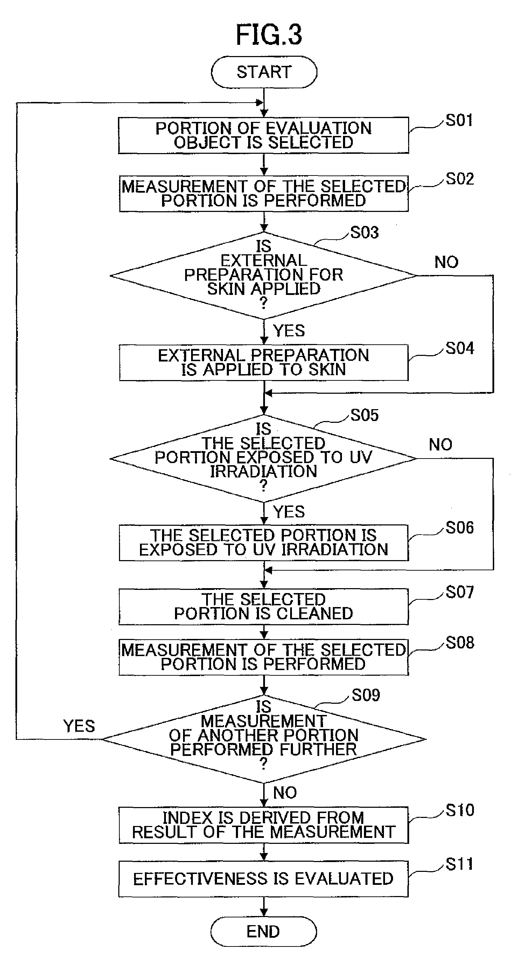

Next, the effectiveness evaluating method of this embodiment which evaluates the effectiveness of an external preparation for skin for protection against ultraviolet irradiation will be described. FIG. 3 is a flowchart for explaining the effectiveness evaluation method of this embodiment which evaluates the effectiveness of an external preparation for skin for protection against ultraviolet irradiation.

In the flowchart illustrated, the effectiveness evaluation method is performed so that depth distributions of the amount of urocanic acid in the portion of the evaluation object both before and after application of the external preparation to the skin and both before and after exposure to ultraviolet irradiation, which are required as measurement conditions in this embodiment, can be measured.

Upon start of the effectiveness evaluation method illustrated in FIG. 3, a portion of the evaluation object (one of skin of bodily parts, such as a hand, an arm, a leg, etc., or a predetermined range of the skin) is selected (S01). Before application of the UV protective external preparation, the measurement of the selected portion is performed (S02). In the step S02, a depth distribution of the amount of urocanic acid in the arbitrarily selected portion of the evaluation object is measured by Raman spectroscopy.

Subsequently, it is determined whether the external preparation for skin is applied (S03). When it is determined in the step S03 that the external preparation is applied, an external preparation for skin for protection against ultraviolet irradiation that is set up beforehand for the evaluation object is applied (S04). When applying the external preparation, the external preparation for skin for protection against ultraviolet irradiation is applied to an area of 30 square centimeters at a rate of 2 mg per square centimeter, for example. The amount of the external preparation applied should be in conformity with the requirements specified beforehand in a corresponding SPF measuring method for general standard samples (for example, those specified in "Cosmetics for protection against ultraviolet irradiation and Ultraviolet Irradiation Protective Effects - SPF and PA indication - 2003 revised version" from Japan Cosmetic Industry Association). However, it is to be understood that the foregoing description is exemplary and explanatory and is not restrictive of the amount of the external preparation applied according to the invention as claimed.

After the external preparation is applied, it is preferred to let the external preparation stand for a predetermined time such that an appreciable effect is presented. Although the time to let the external preparation stand varies depending on the kind of the external preparation, the amount of the external preparation applied, the applied portion, etc., the preferred time is about 15 minutes or 30 minutes.

When it is determined in the step S03 that the external preparation is not applied or after the step S04 is completed, it is determined whether the selected portion in the step S01 is exposed to ultraviolet irradiation (S05).

When it is determined in the step S05 that the selected portion is exposed to ultraviolet irradiation, the selected portion is exposed to solar ultraviolet irradiation simulation light, for example (S06). As for the light irradiated in the step S06, the skin is exposed to, for example, the whole region of ultraviolet light using a solar ultraviolet irradiation simulator (for example, the 601 model solar ultraviolet irradiation simulator with 6 ports, manufactured by Solar Light Company).

The dose of ultraviolet irradiation is measured by using the existing photometer (for example, the 3D-601 model photometer that is dedicated for the above-mentioned simulator). The portion to which the external preparation is not applied can also be exposed to the solar ultraviolet irradiation simulation light. Each of the exposure conditions in this embodiment varies depending on the measurement conditions, the evaluation object portion, the kind or the amount of the external preparation applied, the content of the index, etc. The exposure will be described later with reference to each of the respective evaluation examples.

When it is determined in the step S05 that the selected portion is not exposed to ultraviolet irradiation or after the step S06 is completed, the selected portion is cleaned to eliminate the applied external preparation (S07). When the result of the determination in the step S03 is negative and the external preparation is not applied, performance of the cleaning is optional and may be omitted.

For example, the cleaning is performed as follows. Using a dedicated cleansing cream (for example, the oil cleansing cream dedicated for the sunscreen) the cleaning of the selected portion is performed sufficiently, and thereafter the cleaning is further performed using soap so that the applied external preparation is eliminated from the selected portion.

After the step S07 (cleaning) is completed, measurement of the selected portion is performed (S08).

In this case, a depth distribution of the amount of urocanic acid in the portion that is the same as the portion of the evaluation object whose depth distribution before application of the external preparation has already been performed is similarly measured by Raman spectroscopy. In the step S08, the measurement of the depth distribution may be performed for any of "the portion after application of the external preparation and after exposure to the solar ultraviolet irradiation simulation light", "the portion after application of the external preparation and before exposure to the solar ultraviolet irradiation simulation light", and "the portion before application of the external preparation and after exposure to the solar ultraviolet irradiation simulation light".

At this time, the cleaning of the portion before application of the external preparation and after exposure to the solar ultraviolet irradiation simulation light is performed in the step S07 similarly. After the cleaning of the portion is performed using the dedicated oil cleansing cream and soap, a depth distribution of the amount of urocanic acid in the portion the same as the portion of the evaluation object whose depth distribution before exposure to the solar ultraviolet irradiation simulation light has already been performed is also similarly measured by Raman spectroscopy.

Subsequently, it is determined whether the measurement of another portion of the evaluation object is performed further (S09). In this case, the measurement of another portion may be performed further. Alternatively, the measuring conditions for the same portion may be changed to different ones. Namely, the measuring conditions with respect to the application of the external preparation to the skin (S03) or the exposure to ultraviolet irradiation (solar ultraviolet irradiation simulation light) (S05) may be changed to different ones from the previously selected measuring conditions, and the measurement of the same portion may be performed under the changed measuring conditions.

When it is determined in the step S09 that the measurement of another portion is performed further, the control is returned to the step S01, and subsequent processing of the measurement of another portion of the evaluation object may be performed, or the measurement of the same portion may be performed under different measuring conditions. When it is determined in the step S09 that the measurement of another portion is not performed further, at least one of a total amount of urocanic acid in the horny cell layer, an average amount of urocanic acid in the horny cell layer, a depth distribution of the amount of urocanic acid in the horny cell layer from a skin surface, a residual ratio of urocanic acid in the horny cell layer, and a total amount of urocanic acid at three surface points in the depth distribution is computed as the index based on the measurement results (the amount of urocanic acid in the horny cell layer) (S10). Derivation of the index may be performed by using the index that is set up beforehand, and the index may be selected by taking into consideration the portion of the evaluation object, the components of the external preparation for skin, or the contents of the previously computed index having been used for evaluation.

Subsequently, the effectiveness of the external preparation for protection against ultraviolet irradiation is evaluated based on the acquired index (S11). Specifically, the step S11 evaluates the effectiveness of the external preparation for protection against ultraviolet irradiation by comparing the depth distributions of the amount of urocanic acid in the horny cell layer.

Accordingly, the effectiveness of an external preparation for skin for protection against ultraviolet irradiation can be evaluated simply and speedily based on the index derived from the depth distributions of the amount of urocanic acid in the skin measured by Raman spectroscopy. Specifically, the effectiveness of a sun screen material can be evaluated with high precision based on the index derived from the amount of cis-urocanic acid or the trans/cis ratio obtained by the confocal Raman microspectroscopy, for example.

### <Example 1 of Index Derivation and Evaluation: S10>

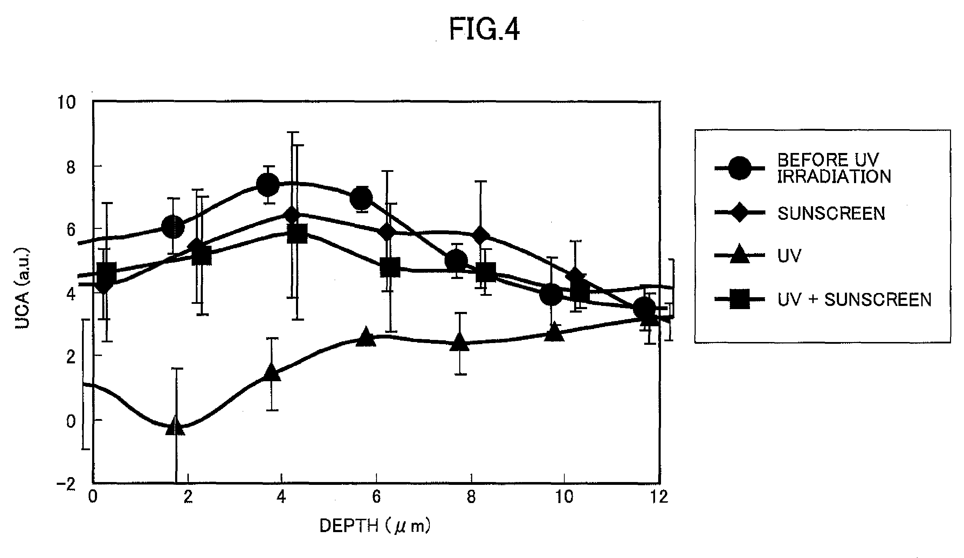

Next, an example of the index derivation and evaluation will be described. FIG. 4 is a diagram for explaining Example 1 of the index derivation and evaluation in this embodiment. Namely, an example of the index obtained by using the depth distributions of the amount of urocanic acid in the horny cell layer measured by Raman spectroscopy is illustrated in FIG. 4.

In FIG. 4, the horizontal axis indicates the depth (in micrometer), and the vertical axis indicates the amount of urocanic acid (UCA (a.u.)) in a horny cell layer. The measurement results of the amount of urocanic acid in the horny cell layer for each of the measuring conditions "Before UV Irradiation", "UV", "Sunscreen" and "UV + Sunscreen" are plotted.

As illustrated in FIG. 4, the values of the amount of urocanic acid in the horny cell layer for every depth of 2 micrometers in the depth distribution measured by Raman spectroscopy are plotted, and by looking at a difference in the amount of urocanic acid between before and after exposure to the solar ultraviolet irradiation simulation light at each point, it is detectable which of the several depths in the skin has been reached by the ultraviolet irradiation.

For example, at the time of evaluation, the comparison of the depth distributions of the amount of urocanic acid in the horny cell layer between before and after the application of the external preparation to the skin is performed based on how much and to which depth the urocanic acid remains. Based on the result of the above-described comparison, the effectiveness of an external preparation for skin for protection against ultraviolet irradiation can be evaluated.

### <Example 2 of Index Derivation and Evaluation: S10>

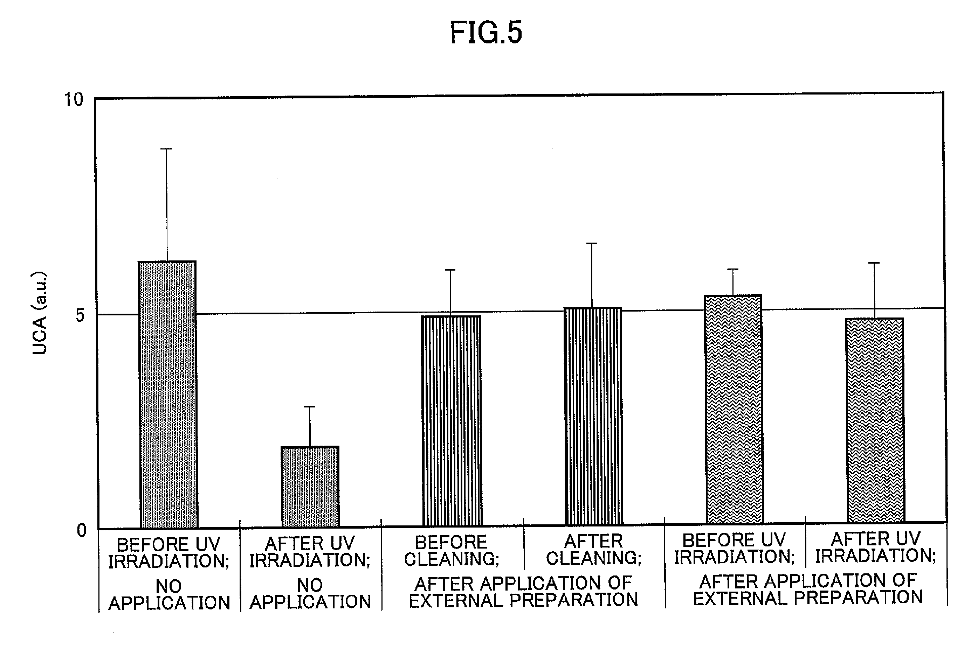

Next, another example of the index derivation and evaluation other than the above Example 1 will be described. FIG. 5 is a diagram for explaining Example 2 of the index derivation and evaluation in this embodiment.

Unlike the Example 1 of the index derivation and evaluation mentioned above, in the Example 2 of the index derivation and evaluation, as illustrated in FIG. 5, an average amount of urocanic acid in the entire thickness of the horny cell layer is used as the index for evaluation. In FIG. 5, the average amount of urocanic acid (UCA (a. u.)) in the horny cell layer for each of the respective cases "Before UV Irradiation and no Application", "After UV Irradiation and no Application", "Before Cleaning and After Application of External Preparation", "After Cleaning and After Application of External Preparation", "Before UV Irradiation and After Application of External Preparation", and "After UV Irradiation and After Application of External Preparation" is illustrated.

Because the amount of urocanic acid measured by Raman spectroscopy is plotted for every depth of 2 micrometers in the depth distribution, the average amount of urocanic acid is represented by the average of the amounts of urocanic acid at seven surface points (0 micrometer, 2 micrometers, 4 micrometers, 6 micrometers, 8 micrometers, 10 micrometers, 12 micrometers) in the depth distribution from the skin surface to the depth of 12 micrometers.

Accordingly, the effectiveness of an external preparation for skin for protection against ultraviolet irradiation can be evaluated by using the average amount of urocanic acid in the horny cell layer as the index.

### <Example 3 of Index Derivation and Evaluation: S10>

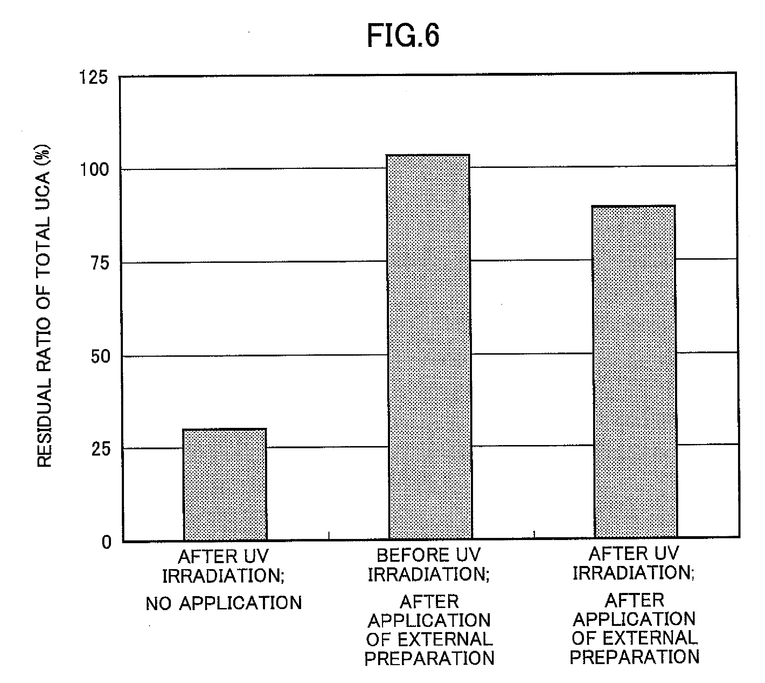

Next, another example of the index derivation and evaluation other than the above Examples 1 and 2 will be described. FIG. 6 is a diagram for explaining Example 3 of the index derivation and evaluation in this embodiment.

Unlike the above two examples, in the Example 3 of the index derivation and evaluation, as illustrated in FIG. 6, a residual ratio of a total amount of urocanic acid in a horny cell layer is used as the index for evaluation. In FIG. 6, the residual ratio (%) of the total amount of urocanic acid in the horny cell layer for each of the respective cases "After UV Irradiation and no Application", "Before UV Irradiation and After Application of External Preparation", and "After UV Irradiation and After Application of External Preparation" is illustrated.

The total amount of urocanic acid in the horny cell layer is represented by a sum of the amounts of urocanic acid for every depth of 2 micrometers in the depth distribution measured by Raman spectroscopy for the whole thickness of the horny cell layer.

Accordingly, the effectiveness of an external preparation for skin for protection against ultraviolet irradiation can be evaluated by using the total amount of urocanic acid in the horny cell layer as the index.

### <Example 4 of Index Derivation and Evaluation: S10>

Next, another example of the index derivation and evaluation other than the above three examples will be described. Unlike the above three examples, in the Example 4 of the index derivation and evaluation, a total amount of urocanic acid at three surface points in the depth distribution is used as the index for evaluation.

Because the amount of urocanic acid measured by Raman spectroscopy is plotted for every depth of 2 micrometers in the depth distribution, the total amount of urocanic acid at the three surface points is represented by a sum of the amounts of urocanic acid (2 micrometers, 4 micrometers, 6 micrometers) of the three surface points in the depth distribution from the depth of 2 micrometers to the depth of 6 micrometers. Accordingly, the effectiveness of an external preparation for skin for protection against ultraviolet irradiation can be evaluated by using the total amount of urocanic acid at three surface points in the depth distribution as the index.

In each of the above Examples 1 to 4 of the index derivation and evaluation, at least one index is derived. Two or more of the Examples 1 to 4 of the index derivation and evaluation may be combined and used for the evaluation of the effectiveness.

### <Examples of Effectiveness Evaluation>

Next, several examples of the effectiveness evaluating method which evaluates the effectiveness of an external preparation for skin for protection against ultraviolet irradiation based on the index derived from the results of the measurement will be described.

In the following, in the evaluation of the effectiveness of the external preparation for skin for protection against ultraviolet irradiation, the dose of solar ultraviolet irradiation simulation light and the SPF of the external preparation are varied, the depth distributions of the amount of urocanic acid in the horny cell layer both before and after application of the external preparation and both before and after exposure to the solar ultraviolet irradiation simulation light are measured by Raman spectroscopy, and the effectiveness of the external preparation is evaluated based on the measured depth distributions of the amount of urocanic acid in the horny cell layer.

### <Example 1>

A first example (Example 1) of the effectiveness evaluating method will be described. In the Example 1, in the state where the external preparation is not applied, the residual ratios of urocanic acid in the horny cell layer before and after exposure to the solar ultraviolet irradiation simulation light are compared.

The dose of solar ultraviolet irradiation simulation light (for example, by the solar ultraviolet irradiation simulator manufactured by Solar light Company) is changed to one of the erythema UV luminous intensities of 1MED/min/cm2, 2MED/min/cm2, and 3MED/min/cm2, which are in the unit of intensity of the ultraviolet irradiation of the above-mentioned simulator, each measured using the photometer (3D-601) dedicated for the above-mentioned simulator. The results of the measurement are compared.

Before exposure to the solar ultraviolet irradiation simulation light, the amount of urocanic acid in the horny cell layer is measured by the Raman method. Moreover, after exposure to the solar ultraviolet irradiation simulation light, a depth distribution of the amount of urocanic acid in the portion that the same as the portion of the measurement object before the application of the external preparation is similarly measured by Raman spectroscopy.

Under the above measuring conditions, the depth distributions of the amount of urocanic acid in the horny cell layer both before and after the application of the external preparation to the skin and both before and after the exposure to the solar ultraviolet irradiation simulation light are measured by Raman spectroscopy, respectively.

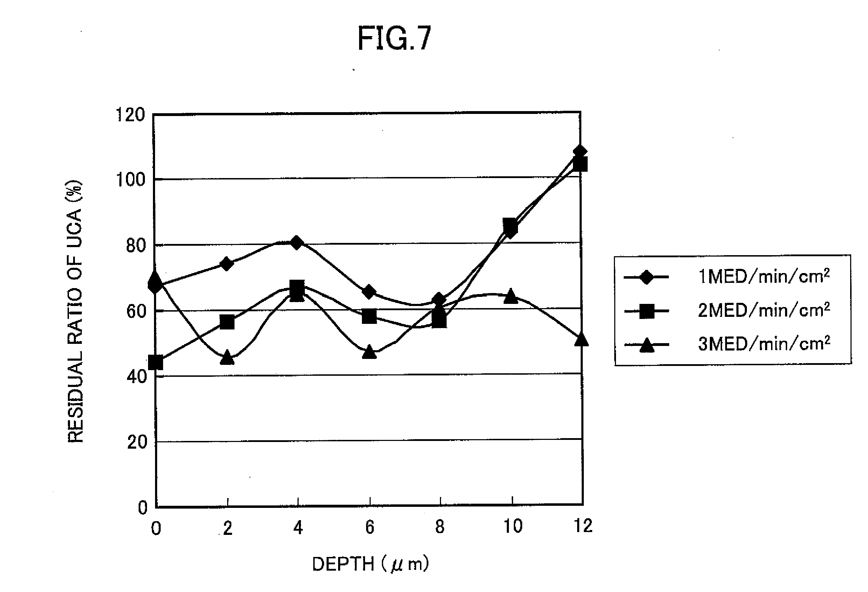

FIG. 7 is a diagram illustrating an example of the evaluation result by the Example 1. In FIG. 7, the horizontal axis indicates the depth (in micrometers), and the vertical axis indicates the residual ratio (%) of the total amount of urocanic acid (UCA) in the horny cell layer. The notation "MED/min/cm2" in FIG. 7 is the unit of the measurement value measured by the photometer dedicated for the above-mentioned solar ultraviolet irradiation simulator (3D-601) by Solar Light Company, which is the unit of luminous intensity per square centimeter, which will be referred to as the erythema UV luminous intensity.

At this time, the residual ratio of the total amount of urocanic acid (UCA) in the horny cell layer is 76.0% when exposed to the erythema UV luminous intensity 1MED/min/cm2, 66.0% when exposed to the erythema UV luminous-intensity 2MED/min/cm2, and 57.4% when exposed to the erythema UV luminous-intensity 3MED/min/cm2. Therefore, it is found out that the residual ratio of the total amount of urocanic acid (UCA) in the horny cell layer depends on the dose of solar ultraviolet irradiation simulation light. The skin state at this time is as follows. When exposed to 1MED/min/cm2, the erythema is not present, and when exposed to 2MED/min/cm2, the erythema is present. It is found out that the erythema UV luminous-intensity 2MED/min/cm2 is the minimal erythema dose for this test subject.

Specifically, FIG. 7 illustrates a change of the plots of the amount of urocanic acid in the depth direction depending on the dose of solar ultraviolet irradiation simulation light for the purpose of evaluation. As illustrated in FIG. 7, when exposed to the solar ultraviolet irradiation simulation light that is above the minimal erythema dose (the erythema UV luminous-intensities 2MED/min/cm2 and 3MED/min/cm2), the residual ratio in the range of the depths of 0-8 micrometers is not so greatly changed. When exposed to the solar ultraviolet irradiation simulation light that is below the minimal erythema dose (the erythema UV luminous-intensity 1MED/min/cm2), the residual ratio is changed greatly.

It is found out from the results of the measurement that the residual ratio of urocanic acid changes in proportion to the dose of solar ultraviolet irradiation simulation light. If the residual ratio of urocanic acid when exposed to a small amount of ultraviolet irradiation that does not cause the erythema to take place in the skin is measured, a new index for evaluation of the adverse effects of ultraviolet irradiation on the skin which is more sensitive than the erythema can be obtained, and the osmosis of ultraviolet irradiation to the skin can be detected suitably.

Thus, by using the above-described Examples, the effectiveness evaluation can be carried out without damaging the skin, and the above-described Examples of the effectiveness evaluation do not require tape stripping or extraction using a potassium hydroxide solution as in the conventionally reported methods.

The conventional methods have to extract urocanic acid from the skin and measure the amount of urocanic acid outside the skin, but the effectiveness evaluating method of this embodiment can measure the amount of urocanic acid within the skin. The isomerization of urocanic acid at a certain depth in the skin can be detected in the same position before and after exposure to UV irradiation.

The effectiveness evaluating method of this embodiment does not require the time for processing of samples and is able to evaluate the effectiveness of the external preparation for protection against UV irradiation simply and speedily. Specifically, performing one measurement takes about 5 minutes only and the total time for measurement is about 30 minutes including 15 minutes of warm-up time after application of a UV protective external preparation for skin. It is confirmed that, even when the external preparation is applied to the skin and the skin is cleaned using the dedicated cleansing cream and soap together, the residual urocanic acid in the horny cell layer is not eliminated by the cleaning.

### <Example 2>

Next, a second example (Example 2) of the effectiveness evaluating method will be described. In the Example 2, the residual ratios of urocanic acid in the horny cell layer before and after application of the external preparation are compared between before and after exposure to the solar ultraviolet irradiation simulation light, and the effectiveness is evaluated.

The UV protective external preparation for skin used is the model sunscreen of SPF30, PFA10. The external preparation is applied to an area 30 cm2 at a rate of 2 mg/cm2 according to the application method of the SPF measuring method. The dose of solar ultraviolet irradiation simulation light is in the amount of ultraviolet irradiation of the erythema UV luminous-intensity 1MED/min/cm2. For the measurement test, 18 healthy male persons (the average age: 37.1 years old) have been picked up and the forearm part of each person has been tested.

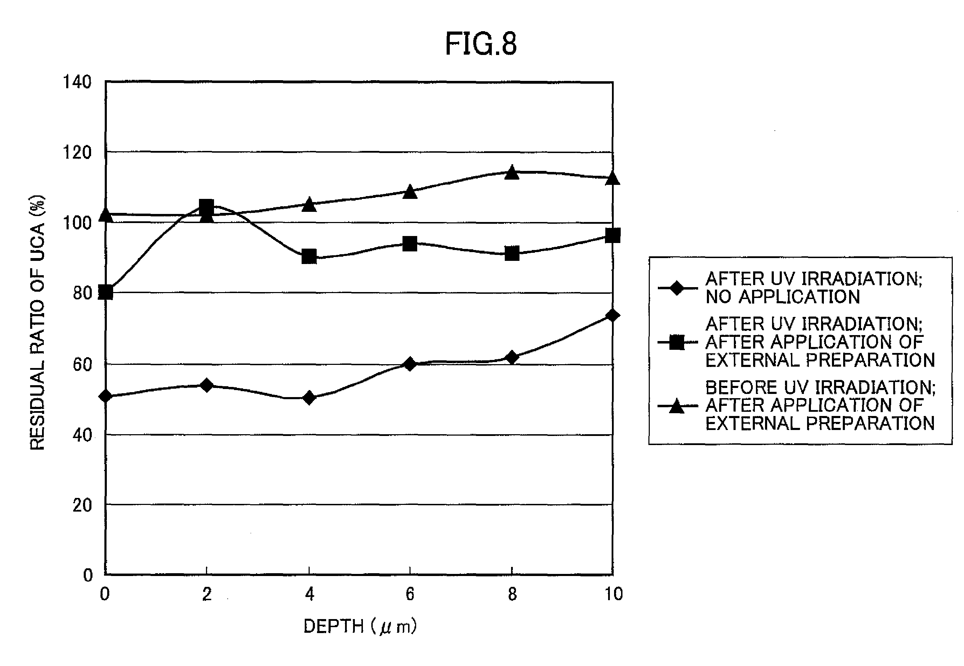

Under the above measuring conditions, the depth distributions of the amount of urocanic acid in the horny cell layer for before and after application of the external preparation and for before and after exposure to the solar ultraviolet irradiation simulation light are measured by Raman spectroscopy, respectively. FIG. 8 is a diagram illustrating an example of the evaluation result by the Example 2. In FIG. 8, the horizontal axis indicates the depth (in micrometers), and the vertical axis indicates the residual ratio (%) of the total amount of urocanic acid (UCA) in the horny cell layer. The evaluation result for each of the respective cases "After UV Irradiation and No Application", "After UV Irradiation and After Application of External Preparation", and "Before UV Irradiation and After Application of External Preparation" is illustrated in FIG. 8.

Specifically, FIG. 8 illustrates a change of the plots of the amount of urocanic acid in the depth direction depending on the application of the external preparation for the purpose of evaluation. As illustrated in FIG. 8, when exposed to the solar ultraviolet irradiation simulation light of the erythema UV luminous-intensity 1MED/min/cm2, in the state in which the external preparation is not applied, the residual ratio of urocanic acid at each point over the whole region of the horny cell layer is in a range of 40 to 50%.

However, after the external preparation is applied and the skin is exposed to the solar ultraviolet irradiation simulation light, the isomerization of urocanic acid takes place only at the outermost points of the skin, and the isomerization is not present at other points of the skin. It is found out that the ultraviolet irradiation does not reach the lower part of the horny cell layer and the external preparation applied is effective for protection against ultraviolet irradiation.

Moreover, it is found out that the isomerization of urocanic acid in the horny cell layer does not occur even when the external preparation after the application to the skin is cleaned using the dedicated cleansing cream and soap together.

### <Example 3>

Next, a third example (Example 3) of the effectiveness evaluating method will be described. In the Example 3, the comparison of UV protective external preparations having different SPF values is performed, and the effectiveness for protection against ultraviolet irradiation is evaluated.

In the Example 3, the comparison between the model sunscreen X of the external preparation of SPF60 and the model sunscreen Y of the external preparation of SPF30 is performed. The dose of solar ultraviolet irradiation simulation light exposure is the erythema UV luminous-intensity 1MED/min/cm2, and a depth distribution of the amount of urocanic acid in the horny cell layer for each of the external preparations having the different SPF values is measured by Raman spectroscopy. When bare skin is exposed to the solar ultraviolet irradiation simulation light used at this time, the erythema is not present. The dose used at this time is below the minimal erythema dose.

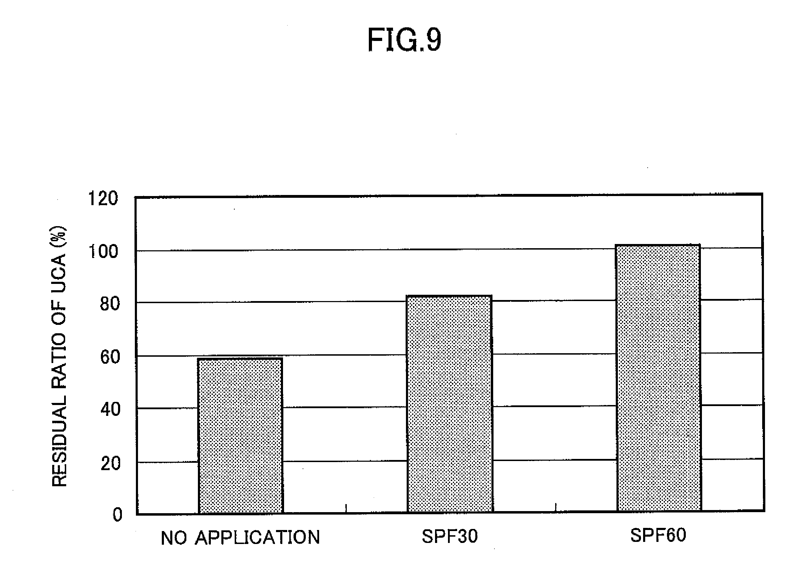

FIG. 9 is a diagram illustrating an example of the evaluation result by the Example 3. In FIG. 9, the residual ratio of urocanic acid (%) for each of the "no application" case, the "SPF30" case, and the "SPF60" case is illustrated. In the analysis of the Example 3, after the values of depth distributions of the amount of urocanic acid in the horny cell layer measured by Raman spectroscopy are plotted, a total amount of urocanic acid at three surface points in each of the depth distributions from the skin surface to the depth of 4 micrometers is computed, and a residual ratio of each total amount of urocanic acid for the three cases is determined as illustrated in FIG. 9.

The result of the comparison of the model sunscreens X and Y shows that the residual ratio of the total urocanic acid at the three surface points in the depth distribution before the application of the external preparation is 58.4%, but the residual ratio for the model sunscreen Y of SPF30 is 81.8%, and the residual ratio for the model sunscreen X of SPF60 is 100.7% as illustrated in FIG. 9.

Thus, even when the UV protective external preparations of SPF30 and SPF60 are exposed to ultraviolet irradiation that is below a minimal erythema dose, the effectiveness for protection against ultraviolet irradiation can be compared. Therefore, it is turned out that the present example provides a new index for evaluating the effectiveness of the external preparation for skin for protection against ultraviolet irradiation.

Two or more of the above Examples 1 to 3 may be combined and executed for practical used, respectively, and the results of the Examples 1 to 3 show that the effectiveness of an external preparation for skin for protection against ultraviolet irradiation can be evaluated by a measured amount of urocanic acid in a horny cell layer.

Therefore, according to the present disclosure, a depth distribution of the amount of urocanic acid in the horny cell layer is measured by Raman spectroscopy both before and after the application of the external preparation to the skin and both before and after exposure to solar ultraviolet irradiation simulation light. By comparing the depth distributions, the effectiveness of the external preparation for skin for protection against ultraviolet irradiation can be evaluated based on the index drawn from the measured values.

As described in the foregoing, according to the present disclosure, the effectiveness of an external preparation for skin with an ultraviolet irradiation protective effect can be simply and speedily evaluated based on the index derived from the depth distributions of the amount of urocanic acid in the skin measured by Raman spectroscopy. Specifically, the effectiveness of the external preparation for skin for protection against ultraviolet irradiation can be evaluated based on the index (for example, a change in the amount of urocanic acid in the horny cell layer) derived from the results of the measurement of the amount of urocanic acid in the horny cell layer and the depth distributions thereof by Raman spectroscopy. More specifically, by using the amount of urocanic acid in the horny cell layer and the depth distributions measured by Raman spectroscopy, the effects of UV irradiation on the skin before and after the application of the external preparation can be evaluated.

Examples of the external preparation for skin which are usable according to the invention include ointment, cream, lotion, spray, pasta, liniment, taping agents, fats and oils, powders, etc.

The present disclosure is not limited to the specifically described embodiments, and it is to be understood that variations and modifications may be made without departing from the scope of the invention as claimed.

This international application is based upon and claims the benefit of priority of Japanese patent application No. 2007-286607, filed on November 2, 2007, the contents of which is hereby incorporated by reference in their entirety.

## Claims

1. A method for evaluating effectiveness of an external preparation for skin for protection against ultraviolet irradiation after the external preparation is applied to the skin, comprising:
a measurement step of measuring an amount of urocanic acid in a horny cell layer by Raman spectroscopy both before and after the application of the external preparation and both before and after exposure to ultraviolet irradiation;
an index derivation step of computing at least one index for evaluating the effectiveness of the external preparation, based on results of the measurement obtained in the measurement step; and
an evaluation step of evaluating the effectiveness of the external preparation by using the at least one index obtained in the index derivation step.

2. The method according to claim 1,
wherein the measurement step measures a depth distribution of the amount of urocanic acid in the horny cell layer both before and after the application of the external preparation.

3. The method according to claim 1,
wherein the index derivation step computes at least one of a total amount of urocanic acid in the horny cell layer, an average amount of urocanic acid in the horny cell layer, a depth distribution of the amount of urocanic acid in the horny cell layer from a skin surface, a residual ratio of urocanic acid in the horny cell layer, and a total amount of urocanic acid at three surface points in the depth distribution, as the at least one index based on the results of the measurement.

4. An effectiveness evaluating device which evaluates effectiveness of an external preparation for skin for protection against ultraviolet irradiation after the external preparation is applied to the skin, comprising:
a measurement part to measure an amount of urocanic acid in a horny cell layer by Raman spectroscopy both before and after the application of the external preparation and both before and after exposure to ultraviolet irradiation;
an index derivation part to compute at least one index for evaluating the effectiveness of the external preparation, based on results of the measurement obtained by the measurement part; and
an evaluation part to evaluate the effectiveness of the external preparation by using the at least one index obtained by the index derivation part.

5. The effectiveness evaluating device according to claim 4, wherein the measurement part measures a depth distribution of the amount of urocanic acid in the horny cell layer both before and after the application of the external preparation.

6. The effectiveness evaluating device according to claim 4, wherein the index derivation part computes at least one of a total amount of urocanic acid in the horny cell layer, an average amount of urocanic acid in the horny cell layer, a depth distribution of the amount of urocanic acid in the horny cell layer from a skin surface, a residual ratio of urocanic acid in the horny cell layer, and a total amount of urocanic acid at three surface points in the depth distribution, as the at least one index based on the results of the measurement.

7. An effectiveness evaluating program which evaluates effectiveness of an external preparation for skin for protection against ultraviolet irradiation after the external preparation is applied to the skin, and, when executed by a computer, causes the computer to perform:
a measurement step of measuring an amount of urocanic acid in a horny cell layer both before and after the application of the external preparation by Raman spectroscopy, which amount has changed by exposure to ultraviolet irradiation;
an index derivation step of computing at least one index for evaluating the effectiveness of the external preparation, based on results of the measurement obtained in the measurement step; and
an evaluation step of evaluating the effectiveness of the external preparation by using the at least one index obtained in the index derivation step.
